# EUROPÄISCHE PATENTANMELDUNG

(11) **EP 1 980 863 A1**
(43) Veröffentlichungstag der Anmeldung: **15.10.2008**
(21) Anmeldenummer: 06844004.9
(22) Anmeldetag: 12.12.2006
(51) Int. Cl.: G01R 27/26

(54) **EINRICHTUNG ZUM KONTAKTLOSEN DETEKTIEREN BRENNBARER UND EXPLOSIVER FLÜSSIGKEITEN**

(30) Priorität: 20.01.2006 RU 2006101601
(71) Anmelder: Korzhenevsky, Aleksander Vladimirovich, Moscow 117321 (RU)
(72) Erfinder: Korzhenevsky, Aleksander Vladimirovich, Moscow 117321 (RU)
(74) Vertreter: Jeck, Anton
(86) Internationale Anmeldenummer: PCT/RU2006/000663
(87) Internationale Veröffentlichungsnummer: WO 2007/084025

(57) **Zusammenfassung**

Die Erfindung betrifft eine Einrichtung zum kontaktlosen Nachweis brennbarer und explosiver Flüssigkeiten mit einem Gehäuse, einem Alarmanzeiger, einer speziellen Recheneinrichtung und einer Wechselstromversorgung. Der Prüfbereich und die Funktionen werden **dadurch** erweitert und die Bedienungsfreundlichkeit erhöht, dass Elektroden in einer Reihe auf einer Seite des Gehäuses so angeordnet sind, dass daran außerhalb des Gehäuses Potentiale messbar sind, dass eine in der Reihe außen liegende Elektrode als Außenelektrode mit der Wechselstromversorgung verbunden ist, dass die übrigen Elektroden als Messelektroden über Spannungsmesser mit Eingängen der speziellen Recheneinrichtung verbunden sind und dass die spezielle Recheneinrichtung so ausgebildet ist, dass sie die Kennliniensteilheit der Spannungsverteilung an den Messelektroden misst, den gemessenen Wert der Steilheit mit einem vorgegebenen, maximalen Wert der Steilheit für eine nicht feuergefährliche Flüssigkeit vergleicht und bei der Überschreitung des Wertes der Steilheit bezüglich des vorgegebenen Wertes der Steilheit für eine nicht feuergefährliche Flüssigkeit ein Alarmsignal über den an einem Ausgang der speziellen Recheneinrichtung angeschalteten Alarmanzeiger abgibt.

## Beschreibung

Die Erfindung betrifft eine Einrichtung zum kontaktlosen Nachweis brennbarer und explosiver Flüssigkeiten mit einem Gehäuse und einem daran angebrachten Alarmanzeiger, der für eine Warnung bei feuergefährlicher Flüssigkeit geeignet ist, mit einer speziellen Recheneinrichtung, die im Gehäuse untergebracht ist und mit einem ihrer Ausgänge am Eingang des Alarmanzeigers angeschlossen ist, und mit einer Wechselstromversorgung.

Die Erfindung gehört zu den technischen Mitteln zur Ermittlung der physischen Eigenschaften von Flüssigkeiten. Sie kann bei der Kontrolle und für die Gewährleistung der Sicherheit auf den Flughäfen, Checkpoints, in Orten mit Menschenanhäufung (Stadien, Schulen, Diskotheken) verwendet werden.

Bekannt ist eine Einrichtung zur Sichtbarmachung der Raumverteilung der dielektrischen Durchlässigkeit innerhalb der Objekte. Die Einrichtung wird Elektrokapazität-Tomograph genannt [Reinecke N. and Mewes D. 1996, Recent developments and industrial/research applications of capacitance tomography, Meas. ScL Technol. 7, S. 233-246]. In der Einrichtung werden Mehrelektroden-Kapazitätssysteme verwendet, die aus Dutzenden von Elektroden bestehen. Die Elektroden umgeben das Objekt von allen Seiten. Die Bilder werden nach den Messergebnissen durch die Lösung der Rückaufgabe für eine Gleichung erneuert. Die Gleichung beschreibt das elektrische Feld in einem inhomogenen Milieu. Für die Prüfung der Flüssigkeit auf Feuergefährlichkeit werden die Elektrokapazität-Tomographen nicht verwendet, weil sie sehr komplex und teuer sind, und weil es notwendig ist, das zu untersuchende Objekt ganz ins Messgerät zu setzen. Die Einrichtung kann für die elektroleitenden Milieus nicht verwendet werden.

Eine Einrichtung zur kontaktlosen Prüfung der Flüssigkeit in einem Gefäß in Bezug auf Feuergefährlichkeit liegt der Erfindung nach ihrer Funktion besonders nahe. Die Einrichtung besteht aus einem Gehäuse, einem Alarmanzeiger am Gehäuse, der für die Meldung über eine feuergefährliche Flüssigkeit geeignet ist, und einer speziellen

Recheneinrichtung, die sich im Gehäuse befindet, und einer der Ausgänge am Eingang des Alarmanzeigers angeschlossen ist, aus einer Wechselstromversorgung, die im Gehäuse oder außerhalb des Gehäuses angeordnet ist, abhängig von der Ausführung der Einrichtung - stationär oder transportabel, tragbar ist.

Die Funktion dieser Einrichtung beruht auf Mikrowellen-Dielkometrie, im Rahmen welcher dielektrische Durchlässigkeit oder elektrische Leitfähigkeit der Flüssigkeit in einem Gefäß gemessen wird. Die Einrichtung hat einen Höchstfrequenzwellen-Sender mit einer Antenne, Höchstfrequenzwellen-Empfänger mit einer Antenne. Der Empfänger ist an die Einrichtung zur Beurteilung über die Feuergefährlichkeit einer Flüssigkeit angeschlossen. Diese Einrichtung ist mit einer speziellen Recheneinrichtung ausgebildet, wobei einer der Ausgänge zum Alarmanzeiger, und der andere zum Display führt.

Der größte Nachteil der bekannten Einrichtung besteht in der Abhängigkeit der Testergebnisse vom Material und der Stärke der Wandung der Gefäße. Deswegen kann die Einrichtung nur für die Flüssigkeiten verwendet werden, die in dünnwandigen Kunststoffflaschen eingebracht sind. Die Nachteile der Einrichtung bestehen auch darin, dass sie große Ausmaße hat, komplex und teuer ist.

Es ist Aufgabe der Erfindung, den Prüfbereich zu erweitern und die Bedienungsfreundlichkeit zu erhöhen, die Funktionen zu erweitern und die Ausmaße der Einrichtung zu vereinfachen und zu reduzieren.

Diese Aufgabe wird nach der Erfindung dadurch gelöst, dass Elektroden in einer Reihe auf einer Seite des Gehäuses so angeordnet sind, dass daran außerhalb des Gehäuses Potentiale messbar sind, dass eine in der Reihe außen liegende Elektrode als Außenelektrode mit der Wechselstromversorgung verbunden ist, dass die übrigen Elektroden als Messelektroden über Spannungsmesser mit Eingängen der speziellen Recheneinrichtung verbunden sind und dass die spezielle Recheneinrichtung so ausgebildet ist, dass sie die Kennliniensteilheit der Spannungsverteilung an den Messelektroden misst, den gemessenen Wert der Steilheit mit einem vorgegebenen, maximalen Wert der Steilheit für eine nicht feuergefährliche Flüssigkeit vergleicht und bei der Überschreitung des Wertes der Steilheit bezüglich des vorgegebenen Wertes der Steilheit für eine nicht feuergefährliche Flüssigkeit ein Alarmsignal über den an einem Ausgang der speziellen Recheneinrichtung angeschalteten Alarmanzeiger abgibt.

Die Einrichtung kann anders ausgebildet sein. Dabei ist es zweckmäßig, dass ein Anzeiger am Gehäuse angebracht ist, der für eine Warnung bei nicht feuergefährlicher Flüssigkeit geeignet ist, und dass die spezielle Recheneinrichtung so ausgebildet ist, dass sie ein Signal an den an einem anderen Ausgang der Recheneinrichtung angeschalteten Anzeiger abgibt, wenn der gemessene Wert der Kennliniensteilheit der Spannungsverteilung an den Messelektroden kleiner ist als der vorgegebene, maximale Wert der Steilheit für eine nicht feuergefährliche Flüssigkeit.

Eine Weiterbildung sieht vor, dass ein Anzeiger am Gehäuse angebracht ist, der für eine Warnung beim Fehlen eines Gefäßes mit Flüssigkeit geeignet ist und dass die Recheneinrichtung den Durchschnittswert der Spannungen an den Messelektroden misst und so ausgebildet ist, dass sie ein Signal an den an einem anderen Ausgang der Recheneinrichtung angeschalteten Anzeiger abgibt, wenn der gemessene Durchschnittswert der Spannungen kleiner ist als ein vorgegebener Schwellenwert.

Die Elektroden können außerhalb des Gehäuses untergebracht sein, und das Gehäuse ist aus Metall ausgebildet.

Die Elektroden können auch innerhalb des Gehäuses untergebracht sein. Dabei soll wenigstens ein Teil des Gehäuses, der zu den Elektroden gerichtet ist, dielektrisch ausgebildet sein.

Die Erfindung wird anhand eines in den Zeichnungen dargestellten Ausführungsbeispiels näher erläutert. Es zeigen:
Fig. 1 schematisch eine Einrichtung zum kontaktlosen Nachweis von brennbaren und explosiven Flüssigkeiten,
Fig. 2 die Kenndaten der Spannungsverteilung an den Elektroden für feuergefährliche und nichtfeuergefährliche Flüssigkeiten,
Fig. 3 den Ablaufplan des zusammengefassten Funktionsalgorithmus der speziellen Recheneinrichtung und
Fig. 4 die Bedienungsfreundlichkeit der Einrichtung.

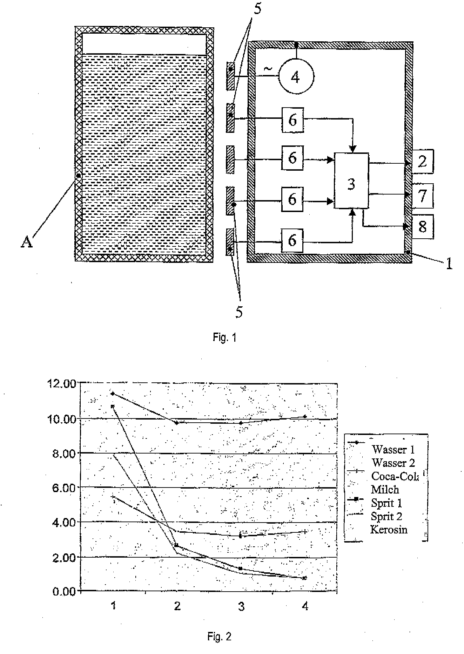

Die Einrichtung zum kontaktlosen Nachweis von brennbaren und explosiven Flüssigkeiten (Fig. 1) hat ein Gehäuse 1 und einen Alarmanzeiger 2, der am Gehäuse 1 angebracht ist. Der Alarmanzeiger 2 ist für die Warnung über die gefährliche Flüssigkeit geeignet. Die spezielle Recheneinrichtung 3 (SRE) ist im Gehäuse 1 untergebracht. Einer der Ausgänge der SRE 3 ist am Eingang des Alarmanzeigers 2 angeschlossen. Die Wechselstromversorgung 4 ist im Gehäuse 1 untergebracht.

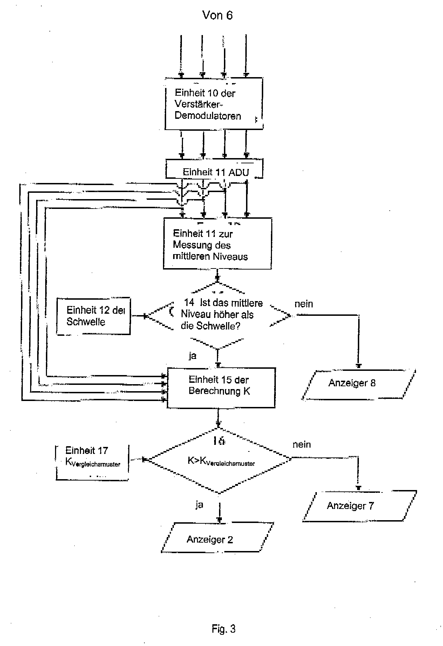

Es werden Elektroden 5 in das Gehäuse 1 eingeführt. Die Elektroden 5 sind in einer Reihe so angebracht, dass an ihnen Potentiale außerhalb des Gehäuses 1 messbar sind. Die in der Reihe außen angeordnete Außenelektrode 5 ist an die Wechselstromversorgung 4 angeschlossen, die restlichen Elektroden 5, als Messelektroden sind an die Spannungsmesser 6 des Spannungswerts angeschlossen. Die Ausgänge der Spannungsmesser 6 sind an die Eingänge der SRE 3 angeschlossen. Die SRE 3 kann die Kennliniensteilheit (Fig. 2) der Spannungsverteilung an den Elektroden 5 messen (wo bei der Abszissenachse die Nummer der Messelektroden gezeigt ist, und an der Ordinatenachse der Wert der gemessenen Spannung an den Messelektroden angegeben ist). Die SRE 3 ist so ausgebildet, dass sie den gemessenen Wert der Steilheit mit der angegebenen Steilheit für eine nicht feuergefährliche Flüssigkeit (Fig. 3) vergleicht. Wenn der gemessene Wert der Steilheit den angegeben Wert der Steilheit überschreitet, ist die SRE 3 so ausgebildet, dass sie für den Anschluss des Alarmanzeigers 2 an einen der Ausgänge der SRE 3 sorgt. Der Alarmanzeiger 2 ist für die Warnung über eine feuergefährliche Flüssigkeit geeignet.

Die Einrichtung kann über einen Anzeiger 7 verfügen. Der Anzeiger 7 ist am Gehäuse 1 angebracht und ist für die Warnung über eine nicht feuergefährliche Flüssigkeit geeignet. Wenn der gemessene Wert der Kennliniensteilheit der Spannungsverteilung an den Elektroden (Fig. 2) kleiner als der angegebene maximale Wert der Steilheit für eine nicht feuergefährliche Flüssigkeit ist, wird die SRE 3 so ausgebildet, dass sie für den Anschluss an einem anderen der Ausgänge (Fig. 1) dieses Anzeigers 7 sorgt.

Die Einrichtung kann über einen Anzeiger 8 verfügen. Der Anzeiger 8 ist am Gehäuse 1 angebracht und ist für die Warnung über das Fehlen des Gefäßes mit Flüssigkeit (Untersuchungsobjekt) in der Nähe des Sensors geeignet. Wenn der gemessene Wert der Spannungen an den Elektroden kleiner als der Sollwert ist, wird die SRE 3 so ausgebildet, dass sie für den Anschluss an einen anderen seiner Ausgänge (Fig. 1) dieses Anzeigers 8 sorgt.

Die Elektroden 5 können außerhalb des Gehäuses 1 angebracht sein, wenn das Gehäuse 1 aus Metall ausgeführt ist (Fig. 1).

Die Elektroden 5 können innerhalb des Gehäuses 1 untergebracht sein. Dabei soll wenigstens ein Teil des Gehäuses 1, der zu den Elektroden 5 gerichtet ist, dielektrisch ausgeführt sein. Dielektrizitätskennzahl ε soll sich der Kennzahl von Luft annähern.

Die Einrichtung funktioniert folgendermaßen.

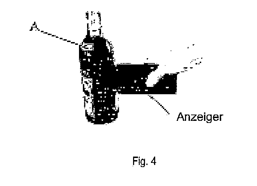

Die Einrichtung wird zum Gefäß A (in Fig. 1, vereinfacht) mit der zu prüfenden Flüssigkeit (Fig. 4) herangetragen, die Wechselstromversorgung 4 wird eingeschaltet. Dabei werden an den Messelektroden 5 Potentiale erzeugt, deren Wert sowohl von der Eingangsspannung der Wechselstromversorgung 4, als auch vom Abstand zwischen der Außenelektrode 5 und den Messelektroden 5 abhängt. Aber es wurde während der Versuche ermittelt, dass unabhängig vom Potential und Abstand zur Außenelektrode 5 und der Leistung der Wechselstromversorgung 4 der Wert der Kennliniensteilheit der Spannungsverteilung an den Elektroden 5 für feuergefährliche Flüssigkeiten (siehe Fig. 2) immer größer ist, als der Wert der Steilheit der Spannungsverteilung an den Elektroden 5 für nicht feuergefährliche Flüssigkeiten. Der Wert der Steilheit hängt kaum vom Material der Wandung des Gefäßes ab, in dem die zu untersuchende Flüssigkeit eingebracht ist. Die Bezeichnungen an den Kurven (Fig. 2) entsprechen folgenden Messbedingungen: Wasser 1 - in Glasflasche ohne Luftspalt; Wasser 2 - in Glasflasche mit Luftspalt 4 mm; Coca-Cola - in Plastikflasche; Milch - in Kartonverpackung Tetra Pak; Sprit 1 - in Glasflasche ohne Luftspalt, Sprit 2 - in Glasflasche mit Luftspalt; Kerosin - in Kartonverpackung für Milch. Aus Fig. 2 ist ersichtlich, dass die Absolutwerte und die räumliche Abhängigkeit der Spannungen an den Elektroden sowohl von den Eigenschaften des Gefäßes als auch vom Luftspalt und den Flüssigkeitseigenschaften abhängen. Unabhängig von den Eigenschaften der Wandung des Gefäßes, erzeugen die Flüssigkeiten mit kleinerer dielektrischer Durchlässigkeit das räumliche Profil mit einer größeren Geschwindigkeit des Abfalls der gemessenen Werte mit dem Abstand von der aktiven Elektrode. Das wird für die Ermittlung der Flüssigkeit unabhängig vom Material und Stärke der Wandung des Gefäßes verwendet.

Die Spannungswerte an den Elektroden 5 (oder der gerichteten Ladung Q₁ - Q₄) an den Ausgängen der Spannungsmesser 6 werden an der SRE 3 verarbeitet. Dafür wird eine Einheit 10 von Verstärker - Demodulatoren verwendet. In diesen werden die Signale verstärkt und zu konstanten Spannungswerten verwandelt. In der Einheit 11 der analogen und digitalen Umformer (ADU) werden die Signale in digitale Form umgewandelt. Die Einheit 12 zur Messung des mittleren Niveaus des Signals ermittelt das mittlere Niveau des eingegangenen Signals. In der Einheit 13 wird die Schwelle für eingehende Signale eingegeben. Die Schwelle wird ausgehend von der Leistung der Wechselstromversorgung 4 und dem Abstand zwischen den Elektroden 5 ausgewählt. Die Einheit 14 des Vergleichs bestimmt, ob das mittlere Niveau des Signals höher als das angegebene Niveau der Schwelle ist. Wenn "nein", dann bedeutet es, dass es kein Untersuchungsobjekt gibt. Nach dem Steuerungsausgang "Nein" schaltet die SRE 3 den Anzeiger 8 ein, der für die Warnung über das Fehlen des Untersuchungsobjekts geeignet ist. Wenn "Ja", dann wird der Steuereingang der Einheit 15 eingeschaltet, und das Signal wird verarbeitet. In der Einheit 15 wird der Wert der Kennliniensteilheit K der Spannungsverteilung an den Elektroden 5 berechnet. Der Wert K wird durch die Einheit 16 des Vergleichs mit K_{Vergleichsmuster} d.h. mit maximaler Steilheit für eine nicht feuergefährliche Flüssigkeit verglichen, die in der Einheit 17 eingegeben ist. Wenn K größer als K_{Vergleichsmuster} ist, ist die Flüssigkeit feuergefährlich. Am Ausgang der Einheit 16 wird "Ja" angezeigt und der Alarmanzeiger 2 eingeschaltet. Wenn K nicht größer als K_{Verglelchsmuster} ist, dann wird am Ausgang der Einheit 16 "Nein" angezeigt und der Anzeiger 7 eingeschaltet. Der Anzeiger 7 ist für die Warnung über eine nicht feuergefährliche Flüssigkeit geeignet. In dieser Einrichtung wird ein Mehrelektrodensystem mit einer linearen Unterbringung der Elektroden 5 verwendet. Es können mindestens 3 und mehr Elektroden ausgewählt werden. Die Einrichtung ermöglicht es, nach der räumlichen Abhängigkeit der gemeinsamen Kapazitäten zwischen der Außenelektrode 5 des Sensors und den restlichen Messelektroden 5 den Einfluss der Wandung des Gefäßes vom Einfluss der Flüssigkeit selbst zu trennen und so die Flüssigkeit richtig und zuverlässig zu prüfen. In der Einrichtung wird ein Vorgehen verwendet, welches nach seinem Sinn der Elektrokapazität-Tomographie nahe kommt. Aber in diesem Fall ist statt der Sichtbarmachung nur die Einschätzung der komplexen dielektrischen Durchlässigkeit einer der Komponenten des heterogenen Milieus notwendig, das aus einem Gefäß, Luftspalt und der Flüssigkeit im Gefäß besteht. Deswegen ist eine geringere Anzahl von Elektroden 5 notwendig. Die gemeinsamen Kapazitäten zwischen den Elektroden 5 können gemessen werden, wobei zwischen einem Paar von Elektroden 5 eine veränderliche Differenz der Potentiale erzeugt wird. Die Außenelektrode 5 wird an die Wechselstromversorgung 4 angeschlossen. Die Ladungen, die durch das elektrische Feld an den restlichen Elektroden 5 entstehen, werden bezüglich einer der aktiven Elektroden 5 oder mit Hilfe eines anderen Verfahrens der Messung der elektrischen Kapazitäten gemessen. Die Elektroden 5 selbst können nur an einer Seite des Objekts (Gefäß mit Flüssigkeit) neben seiner Oberfläche untergebracht werden. So ermöglicht die Einrichtung, die physischen (elektrischen) Eigenschaften der Flüssigkeit unabhängig von der Stärke und dem Material der Wandung des Gefäßes, vom Vorhandensein eines Spalts zwischen dem Mess-System und dem Gefäß zu ermitteln. Dabei sollte man nur den Zugang zu einem kleinen Teil der Oberfläche des Objekts an einer Seite haben. Das unterscheidet die vorgeschlagene Einrichtung positiv sowohl von den Mikrowellen-Dielkometern als auch von den bekannten Elektrokapazität-Tomographen. Es kann mit jeglicher passender Frequenz von Hunderten HZ bis zu 10⁷ HZ gemessen werden Die Kosten, Schwierigkeit und Anforderungen an die Messgeräte sind bei der Ausführung der vorgeschlagenen Einrichtung wesentlich kleiner als bei der Verwendung der Mikrowellen-Dielkometrie (die teuere Versorgung, Empfänger, Antennen für Mikrowellen-Ausstrahlung sind nicht notwendig) oder Kapazität-Tomographie (die Anforderungen an die Anzahl der Kanäle, deren Empfindlichkeit, Recheneinrichtungen für die Datenverarbeitung sind wesentlich kleiner).

Es werden die Funktionen der Einrichtung durch die Messung der Flüssigkeit in Kunststoffgefäßen, sowie in Glasgefäßen (siehe Tabelle) erweitert. Die angemeldete Einrichtung wurde in Form eines Kompaktgeräts ausgeführt. Das Äußere der Einrichtung wird in Fig. 4 gezeigt. Ergebnisse der Tests, die in der Tabelle 5 angeführt sind, zeigen eine hohe Zuverlässigkeit der Untersuchungen von Flüssigkeiten mit Hilfe der vorgeschlagenen Einrichtung. Zusätzlich wird die Konstruktion vereinfacht, und die Ausmaße der Einrichtung werden reduziert.

Die angemeldete Einrichtung zum kontaktlosen Nachweis von brennbaren und explosiven Flüssigkeiten kann erfolgreich für eine operative Ermittlung der eingeführten Flüssigkeiten sowie in den Orten der Menschenanhäufung durch Rechtsschutz- und Sicherheitsorgane eingesetzt werden (gewerbliche Anwendbarkeit).

Vergleichstabelle der mit Hilfe der Einrichtung untersuchten Flüssigkeiten, die in unterschiedlichen Gefäßen untergebracht wurden.

| **Flüssigkeiten** | Ermittelt als | |
|---|---|---|
| | Gefährlich | Gefahrlos |
| Äthylalkohol in Kunststoff- und Glasflasche | + | |
| Sprit in Kunststoff- und Glasflasche | + | |
| Wasser ohne Kohlensäure in Kunststoffflasche | | + |
| Coca-Cola in Kunststoffflasche | | + |
| Milch in Kartonverpackung TetraPak | | + |
| Lösemittel für Emaille in Kunststoffflasche | + | |
| Isopropylalkohol in Glasbehälter | + | |
| Methylalkohol in Glasbehälter | + | |
| Transformatorenöl in Kunststoffbehälter | + | |
| Toluol in Glas | + | |
| Chloroform in Glas | + | |
| Sekt | | + |
| Tee in Kunststoffflasche | | + |
| Diesel in Kunststoffkanister | + | |
| Wodka (40% Alkoholgehalt) | | + |
| Rotwein und Weißwein in Glasflaschen | | + |
| Shampoo | | + |
| Diverse Reinigungsflüssigkeiten für Haushalt | | + |

## Patentansprüche

1. Einrichtung zum kontaktlosen Nachweis brennbarer und explosiver Flüssigkeiten mit einem Gehäuse (1) und einem daran angebrachten Alarmanzeiger (2), der für eine Warnung bei feuergefährlicher Flüssigkeit geeignet ist, mit einer speziellen Recheneinrichtung (SRE, 3), die im Gehäuse (1) untergebracht ist und mit einem ihrer Ausgänge am Eingang des Alarmanzeigers (2) angeschlossen ist, und mit einer Wechselstromversorgung (4),
**dadurch gekennzeichnet,**
**dass** Elektroden (5) in einer Reihe auf einer Seite des Gehäuses (1) so angeordnet sind, dass daran außerhalb des Gehäuses (1) Potentiale messbar sind, dass eine in der Reihe außen liegende Elektrode (5) als Außenelektrode mit der Wechselstromversorgung (4) verbunden ist, dass die übrigen Elektroden (5) als Messelektroden über Spannungsmesser (6) mit Eingängen der speziellen Recheneinrichtung (SRE, 3) verbunden sind und dass die spezielle Recheneinrichtung (SRE, 3) so ausgebildet ist, dass sie die Kennliniensteilheit der Spannungsverteilung an den Messelektroden misst, den gemessenen Wert der Steilheit mit einem vorgegebenen, maximalen Wert der Steilheit für eine nicht feuergefährliche Flüssigkeit vergleicht und bei der Überschreitung des Wertes der Steilheit bezüglich des vorgegebenen Wertes der Steilheit für eine nicht feuergefährliche Flüssigkeit ein Alarmsignal über den an einem Ausgang der speziellen Recheneinrichtung (SRE, 3) angeschalteten Alarmanzeiger (2) abgibt.

2. Einrichtung nach Anspruch 1,
**dadurch gekennzeichnet,**
**dass** ein Anzeiger (7) am Gehäuse (1) angebracht ist, der für eine Warnung bei nicht feuergefährlicher Flüssigkeit geeignet ist, und dass die spezielle Recheneinrichtung (SRE, 3) so ausgebildet ist, dass sie ein Signal an den an einem anderen Ausgang der Recheneinrichtung (SRE, 3) angeschalteten Anzeiger (7) abgibt, wenn der gemessene Wert der Kennliniensteilheit der Spannungsverteilung an den Messelektroden kleiner ist als der vorgegebene, maximale Wert der Steilheit für eine nicht feuergefährliche Flüssigkeit.

3. Einrichtung nach Anspruch 1,
**dadurch gekennzeichnet,**
**dass** ein Anzeiger (8) am Gehäuse (1) angebracht ist, der für eine Warnung beim Fehlen eines Gefäßes (A) mit Flüssigkeit geeignet ist und dass die Recheneinrichtung (SRE, 3) den Durchschnittswert der Spannungen an den Messelektroden misst und so ausgebildet ist, dass sie ein Signal an den an einem anderen Ausgang der Recheneinrichtung (SRE, 3) angeschalteten Anzeiger (8) abgibt, wenn der gemessene Durchschnittswert der Spannungen kleiner ist als ein vorgegebener Schwellenwert.

4. Einrichtung nach Anspruch 1,
**dadurch gekennzeichnet,**
**dass** die Elektroden (5) außerhalb des Gehäuses (1) angeordnet sind und dass das Gehäuse (1) aus Metall besteht.

5. Einrichtung nach Anspruch 1,
**dadurch gekennzeichnet,**
**dass** die Elektroden (5) innerhalb des Gehäuses (1) untergebracht sind und dass wenigstens ein zu den Elektroden (5) gerichteter Teil des Gehäuses (1) dielektrisch ausgebildet ist.
